(19) **Europäisches Patentamt**
**European Patent Office**
**Office européen des brevets**

(11) **EP 2 465 938 A1**

(12) **EUROPEAN PATENT APPLICATION**
published in accordance with Art. 153(4) EPC

(43) Date of publication:
**20.06.2012 Bulletin 2012/25**

(21) Application number: **10808081.3**

(22) Date of filing: **11.08.2010**

(51) Int Cl.:
*C12P 23/00* (2006.01)   *A23K 1/16* (2006.01)
*A23L 1/30* (2006.01)   *A61P 3/00* (2006.01)
*A61K 31/01* (2006.01)   *A61K 31/122* (2006.01)
*A61K 35/74* (2006.01)   *C12R 1/01* (2006.01)
*C12R 1/645* (2006.01)

(86) International application number:
**PCT/JP2010/005036**

(87) International publication number:
**WO 2011/018896 (17.02.2011 Gazette 2011/07)**

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**

(30) Priority: **11.08.2009 JP 2009186206**

(71) Applicant: **Kaneka Corporation
Osaka-shi, Osaka 530-8288 (JP)**

(72) Inventors:
• **INOUE, Hiroaki
Takasago-shi
Hyogo 676-8688 (JP)**

• **KIZAKI, Noriyuki
Takasago-shi
Hyogo 676-8688 (JP)**
• **NANBA, Hirokazu
Takasago-shi
Hyogo 676-8688 (JP)**

(74) Representative: **HOFFMANN EITLE
Patent- und Rechtsanwälte
Arabellastraße 4
81925 München (DE)**

(54) **PRODUCTION METHOD FOR ASTAXANTHIN-CONTAINING COMPOSITION**

(57) The present invention relates to a method for reducing the relative ratio of 3-hydroxy-3',4'-didehydro-$\beta,\psi$-caroten-4-one (HDCO) to astaxanthin in a composition containing astaxanthin and HDCO by contacting the composition with an acidic medium having a pH of 3 or less and/or a basic medium having a pH of 9 or greater, and also relates to a method for producing an astaxanthin-containing composition which includes reducing the relative ratio of HDCO by the above method. By means of the method of the present invention, the relative ratio of HDCO, the biological function of which is not known, in an astaxanthin-containing composition can be easily reduced.

EP 2 465 938 A1

**Description**

TECHNICAL FIELD

The present invention relates to a method for producing an astaxanthin-containing composition. More specifically, the present invention relates to a method for reducing the relative ratio of 3-hydroxy-3',4'-didehydro-$\beta,\psi$-caroten-4-one (HDCO) to astaxanthin in an astaxanthin-containing composition that contains HDCO.

BACKGROUND ART

Astaxanthin is a natural carotenoid, and is widely used as a feed additive for improving the color of .the flesh and skin of farmed fish. Recently, it has also been attracting attention as a material for functional foods.

It is known that astaxanthin is produced in cells of microorganisms of the genera such as *Xanthophyllomyces* (previously known as *Phaffia*)*, Brevundimonas, Haematccoccus, Chlamydomonas, Monoraphidium, Erythrobacter, Agrobacterium,* and *Paracoccus,* and microorganisms of Labyrinthulea. Also, astaxanthin, can be produced by chemical synthesis.

3-Hydroxy-3',4'-didehydro-$\beta,\psi$-caraten-4-one (hereinafter, abbreviated as HDCO) is also a carotenoid, and can be regarded as a by-product of astaxanthin biosynthesis. Since no biological function of HDCO is known, the amount of HDCO in astaxanthin-containing compositions intended for use as, for example, feeds, foods, food additives, and medicaments is preferably reduced relative to the amount of astaxanthin.

However, it has been reported that in the case where a yeast of *Xanthophyllomyces* (previously known as *Phaffia*) is mutated to increase the astaxanthin content, the relative ratio of HDCO to astaxanthin also tends to be increased with the increase of the astaxanthin content (Patent Document 1).

Patent Document 1 discloses a method for producing an astaxanthin-containing composition having a small relative ratio of HDCO/astaxanthin. This method includes selecting a mutant having both of a high astaxanthin content and a low HDCO content from mutant yeast strains of *Xanthopyllomyces* after mutagenesis, and obtaining an astaxanthin-containing composition using the mutant.

Patent Document 1 recites that the selection of the target mutant is carried out by visually distinguishing intensely colored orange colonies which have a high astaxanthin content and a comparatively low ratio of HDCO/astaxanthin, from red colony populations (which have a high astaxanthin content and a comparatively high ratio of HDCO/astaxanthin). However, it is very difficult to select a target strain based only on the color tone of colonies because the red tone of mutants having a lower astaxanthin content is weaker. Therefore, success depends on chance, and a long time and huge efforts are required.

Patent Document 1: Japanese Patent No. 3202018

SUMMARY OF THE INVENTION

The present inventors actually obtained high astaxanthin-content strains as a result of mutagenesis of strains of *Xanthophyllomyces* for the purpose of increasing the astaxanthin content. Since, as shown below, the HDCO amounts of all the high astaxanthin-content strains were more than 10% of the respective astaxanthin amounts, the probability of obtaining such a mutant as in Patent Document 1 is assumed to be very low. Hence, the present invention aims to provide a very simple method by which the relative ratio of HDCO to astaxanthin in an astaxanthin-containing composition that contains HDCO can be reduced.

As a result of intensive studies to solve the above problem, the present inventors have found that the relative ratio of HDCO to astaxanthin in a composition containing astaxanthin and HDCO can be reduced by contacting the composition with an acidic medium having a pH of 3 or less and/or a basic medium having a pH of 9 or greater, and therefore have completed the present invention.

Specifically, the present invention provides a method for reducing a relative ratio of HDCO to astaxanthin in a composition containing astaxanthin and HDCO by contacting the composition with an acidic medium having a pH of 3 or less and/or a basic medium having a pH of 9.or greater. The present invention also provides a method for producing an astaxanthin-containing composition, which includes contacting a composition containing astaxanthin and HDCO with an acidic medium having a pH of 3 or less and/or a basic medium having a pH of 9 or greater to reduce a relative ratio of HDCO to astaxanthin in the composition. The present invention further provides a feed, a food, a food additive, or a medicament which includes an astaxanthin-containing composition obtained by the method.

The present invention makes it possible to efficiently reduce the relative ratio of HDCO to astaxanthin in a composition containing astaxanthin and HDCO. An astaxanthin-containing composition in which the relative ratio of HDCO to astaxanthin is reduced is useful, for example, as a feed, a food, a food additive, or a medicament.

BEST MODE FOR CARRYING OUT THE INVENTION.

The composition containing astaxanthin and HDCO used herein is one containing both the compounds, and components other than these compounds are not particularly limited. The composition may be, for example, a culture of cells capable of simultaneously producing both the compounds, a culture supernatant thereof, the cells, disrupted cells, dried cells, or an extract of the cells. Other examples include partially purified products of these. Compositions containing chemically synthesized astaxanthin are also include in the composition containing astaxanthin and HDCO used herein, as long as they contain HDCO.

Examples of cells capable of simultaneously producing both the compounds include, but are not limited to, cells of microorganisms of the genera such as *Xanthophyllomyces* (previously known as *Phaffia*), *Brevundimonas, Haematocaccus, Chlamydomonas, Monoraphidium, Erythrobacter, Agrobacterium,* and *Paracoccus,* and microorganisms of Labyrinthulea; and mutants, recombinant strains, and self-cloned strains of these microorganisms.

Examples of' microorganisms of Labyrinthulea include microorganisms' of the genera *Thraustochytrium, Schizochytrium,* and *Labyrinthula.*

The acidic medium used herein refers to a liquid material having a pH, as determined by a glass electrode method, of 3 or less, preferably 2 or less, more preferably 1 or less, still more preferably 0.5 or less, and particularly preferably 0.1 or less. The acidic medium can be prepared, for example, by dissolving an acidic material in an appropriate solvent. The acidic material is not particularly limited, as long as the effect of the present invention is successively produced. Examples thereof include inorganic acids such as hydrogen chloride, sulfuric acid, phosphoric acid, and nitric acid; and organic acids such as formic acid and acetic acid. In particular, hydrogen chloride and sulfuric acid are preferred. Any of these acidic materials may be used alone, or any combination thereof may be used.

The basic medium used herein refers to a liquid material having a pH, as determined by a glass electrode method, of 9 or greater, preferably 10 or greater, more preferably 11 or greater, still more preferably 12 or greater, yet still more preferably 13 or greater, even more preferably 13.5 or greater, and particularly preferably 13.9 or greater. The basic medium can be prepared, for example, by dissolving a basic material in an appropriate solvent. The basic material is not particularly limited, as long as the effect of the present invention is successively produced. Examples thereof include hydroxides of alkaline metals and of alkaline earth metals, such as sodium hydroxide, potassium hydroxide, and magnesium hydroxide; and ammonia and gamines. In particular, sodium hydroxide is preferred. Any of these basic materials may be used alone or may be used in combination.

The solvents for dissolving the acidic material and/or the basic material are not particularly limited, as long as the effect of the present invention is successively produced. Examples thereof include water, ethanol, acetone, methanol, and mixed solvents of these. In particular, water is preferred. A solvent used in the composition containing astaxanthin and HDCO may be used as a solvent for the acidic medium and/or the basic medium.

The acidic medium has a pH, as determined by the above method, of 3 or less, preferably 2 or less, more preferably 1, or less, still more preferably 0.5 or less, and particularly preferably 0.1 or less. The acidic medium may contain any compounds other than the acidic material and the solvent, as long as the effect of the present invention is successively produced. The basic medium has a pH, as determined by the above method, of 9 or greater, preferably 10 or greater, more preferably 11 or greater, still more preferably 12 or greater, yet still more preferably 13 or greater, even more preferably 13.5 or greater, and particularly preferably 13.9 or greater. The basic medium may contain any compounds other than the basic material and the solvent, as long as the effect of the present invention is successively produced.

The contact of the composition containing astaxanthin, and HDCO with the acidic medium and/or the basic medium is accomplished by any method, as long as the effect of the present invention is successively produced. For example, th-is may be accomplished by placing the composition containing astaxanthin and HDCO and the acidic medium and/or the basic medium in an appropriate vessel, reactor, or the like, and optionally mixing the contents. The mixing may be carried out in a pipe.

Here, it is preferred that the temperature of the mixture is appropriately controlled. The maximum temperature is typically 140°C, and is preferably 100°C, more preferably 90°C, and still more preferably 70°C. The minimum temperature is typically -20°C, and is preferably 0°C, more preferably 10°C, and still more preferably 30°C. At high temperatures exceeding the maximum temperatures, astaxanthin is likely to be unstable. At low temperatures below the minimum temperatures, the operation tends to be difficult.

The time period for contacting the composition containing astaxanthin and HDCO with the acidic medium or the basic medium is not particularly limited, as long as the effect of the present invention is successively produced. The upper limit of the time period is typically 12000 minutes, and is preferably 1200 minutes, and more preferably 300 minutes. The lower limit thereof is typically 1 minute, and is preferably 10 minutes, and more preferably 60 minutes.

The combination of the pH of the acidic medium and/or the basic medium to be contacted with the composition containing astaxanthin and HDCO, the temperature during the contact treatment, and the time period of the contact treatment is not particularly limited and any combination can be selected as long as the effect of the present invention is successively produced. Specifically, an appropriate combination can be selected based on factors such as the form of the composition containing astaxanthin and HDCO, the type of the acidic medium and/or the basic medium used, the pH of the acidic medium and/or the basic medium used, and the mixing conditions in the contact treatment.

The relative ratio of HDCO to astaxanthin in the composition containing astaxanthin and HDCO can be determined based on a chromatogram of HPLC analysis of the composition at an absorbance of 471 nm. Specifically, the relative ratio can be calculated from the peak area for astaxanthin and the peak area for HDCO by the following equation:

$$\text{(Relative ratio (\%) of HDCO to astaxanthin)} = \{\text{(Peak area for HDCO)}/\text{(Peak area for astaxanthin)}\} \times 100.$$

The HPLC analysis can be carried out, for example, under the following condition.
Column: YMC Carotenoid (4.6 × 250 mm; YMC)
Column temperature: 20°C
Mobile phase: solution A (methanol/methyl-t-butyl ether/1% phosphoric acid aqueous solution = 82/15/3) and solution B (methanol/methyl-t-butyl ether/water/phosphoric acid = 7/90/3/0.03), the mobile phase is run at a flow rate of 1.0 ml/min under the following conditions:

after sample injection,

0 to 30'minutes: 100% solution A;
30 to 90 minutes: linear gradient from 100% solution A to 100% solution B; and
90 to 95 minutes: 100% solution B.

In order to perform the HPLC analysis of the composition containing astaxanthin and HDCO, for example, the composition may be optionally dissolved,in an appropriate solvent and injected into HPLC equipment. Examples of the solvent for dissolving the composition include dimethyl sulfoxide, acetone, chloroform, methylene chloride, methanol, and mixed solvents including any combinations of these. Here, the solvent is not particularly limited, as long as it dissolves the composition. In the case where the composition is a culture of cells capable of producing astaxanthin, the cells, disrupted cells, or the like, that is, the composition contains components insoluble in the solvent, the analysis can be performed, for example, by mechanically disrupting the composition in the solvent to obtain an extract and injecting the extract into the HPLC equipment. The mechanical disruption can be accomplished, for example, by a method using glass beads, or pressure disruption.

The composition containing astaxanthin and HDCO used herein is not particularly limited, as long as it contains both the compounds. The composition may be, for example, a composition having a relative ratio of HDCO/astaxanthin, as determined by the above method, of preferably not less than 1%, and more preferably not less than 5%. In order to achieve the optimum effect of the present invention, the relative ratio of HDCO to astaxanthin is more preferably not less than 10%, and particularly preferably not less than 15%.

As mentioned above, in the case where a microorganism of the genus *Xanthophyllomyces* (previously known as *Phaffia*) is modified to increase the astaxanthin content, the relative ratio of HDCO to astaxanthin also tends to be increased. Hence, the composition containing astaxanthin and HDCO used in the present invention is preferably cells of a microorganism of the genus *Xanthophyllomyces* which have an astaxanthin content of not less than 2000 μg/g dry cell weight, or an astaxanthin-containing composition produced using the cells. The composition is more preferably cells of a microorganism of the genus *Xanthophyl1omyces* which have an astaxanthin content of not less than 3000 μg/g dry cell weight, still more preferably not less than 5000 μg/g dry cell weight, yet still more preferably not less than 8000 μg/g dry cell weight, and particularly preferably not less than 10000 μg/g dry cell weight, or an astaxanthin-containing composition produced using the cells. Regarding such microorganism cells of *Xanthophyllomyces* and such an astaxanthin-containing composition produced using the microorganism cells, the relative ratio of HDCO to astaxanthin is preferably not less than 10%, and more preferably not less than 15%.

In the present invention, the relative ratio (%) of HDCO to astaxanthin, as determined by the above method, is reduced typically by 0.1 percentage points (pp) or more, preferably by 0.5 pp or more, more preferably 1 pp or more, still more preferably 2 pp or more, yet still more preferably 3 pp or more, even more preferably 4 pp or more, and particularly preferably 5 pp or more, through the process of reducing the relative ratio of HDCO to astaxanthin, that is, before and after the contact treatment with the acidic medium and/or the basic medium.
A 1 percentage point (pp) or more reduction means that the value determined by the following formula is 1 or more: .
(Relative ratio (%) of HDCO to astaxanthin in the composition before treatment) - (Relative ratio (%) of HDCO to astaxanthin in the composition after treatment).

An astaxanthin-containing composition prepared in accordance with the present invention, in which the relative ratio of HDCO to astaxanthin is reduced, can be processed into an astaxanthin-containing composition usable as a feed, a food, a food additive, a medicament, or the like, optionally through neutralization of the acidic medium and/or the basic medium, optionally followed by separation from these using common procedures, optionally including rinsing. Such a composition may be further purified and then used for these applications.

For example, in the case where a composition in which the relative ratio of HDCO to astaxanthin is reduced in accordance with the present invention, is soluble in a solvent and the solvent is capable of separating the composition from the acidic medium and/or the basic medium by phase separation, the above common procedures may include separating the acidic medium and/or the basic medium from a solution of the composition dissolved in the solvent, rinsing the resulting solution with water, and evaporating the solvent. On the other hand, in the case where the composition is insoluble in any solvents, the above common procedures include, for example, separating the composition from the acidic medium and/or the basic medium by an operation such as continuous centrifugation or filtration, and optionally rinsing the composition with water. However, the common procedures are not limited to these. An astaxanthin-containing composition obtained as described above can be further processed into a product form suitable as a feed, a food, a food additive, or a medicament. Such a product is also within the scope of the present invention.

The term "feed" used herein is intended to include, but is not limited to, feeds and supplements for aquatic animals such as fish, crustaceans, and shellfish, poultry such as chickens, quails, and ducks, lives tock animals such as cattle, pigs, and sheep, and pets such as dogs and cats, and the like. The term "food" used herein is intended to include, but is not limited to, colorants and supplements as well as diets.

As described above, the present invention makes it possible to efficiently reduce the relative ratio of HDCO to astaxanthin in a composition containing astaxanthin and HDCO, and to efficiently obtain an astaxanthin-containing composition having a smaller content of HDCO mixed, which is suited for use as a feed, a food, a food additive, a medicament, or the like, from a composition containing astaxanthin and HDCO.

EXAMPLES

The following is set forth to more specifically illustrate the present invention by way of examples but is not intended to limit the scope of the present invention.

[Method of extraction for analysis of astaxanthin, and HDCO]

In the case where the composition containing astaxanthin and HDCO is, for example, microorganism cells or the like, a mechanical disruption method using glass beads can be used to obtain an extract containing astaxanthin and HDCO. The extract containing astaxanthin and HDCO obtained by such a method can be subjected to analysis using HPLC equipment.

The composition is placed in a 1.5-ml airtight plastic vessel and centrifuged to recover a precipitate. The precipitate is rinsed with water and centrifuged again. The supernatant is removed, and 1 g of glass beads (diameter 0.5 mm) and 1 ml of acetone are added to the recovered precipitate. The mixture is then subjected to a treatment using a multi-beads shocker (Yasui Kikai Corp.), and the solid residues and the glass beads are removed by filtration through a filter (Cosmonice Filter S, 0.45 $\mu$m, Millipore). In this manner, an acetone solution containing astaxanthin and HDCO extracted is obtained.

The disruption of the composition by the multi-beads shocker includes the steps of 30-second disruption and 30-second cooling (4°C), and these steps are repeated five times each. After checking if the composition is sufficiently disrupted, the acceptable is used for the analysis.

[Relative ratio of HDCO in astaxanthin-producing cells]

*Xanthophyllomyces dendrorhous* NBRC 10129 (obtained from NITE Biological Resource Center, National Institute of Technology and Evaluation located at 2-5-8 Kazusakamatari, Kisarazu-shi, Chiba, Japan, 292-0818) was mutated with N-methyl-N'-nitro-N-nitrosoguandine (NTG), and colonies having a strong red tone grown on an agar plate were selected. Thus, mutants with an elevated astaxanthin content were obtained. The same procedure was repeated on the obtained mutants, thereby obtaining mutants with a further elevated astaxanthin content. In this manner, *Xanthophyllomyces dendrorhous* KNK-01, KNK-02-1, KNK-02-2, and KNK-03, which are mutants with an elevated astaxanthin content, were obtained.

Table 1 shows the astaxanthin content and the relative ratio of HDCO to astaxanthin of the obtained mutants. The mutants were grown as follows: preparing 30 ml of a medium (ammonium phosphate 1.3%, potassium phosphate 0.7%, succinic acid 0.6%, yeast extract 0.3%, pH 5.4) in a 500-ml Sakaguchi flask; sterilizing the medium in an autoclave;

inoculating an astaxanthin-producing strain into the medium; and incubating the medium at 20°C for 180 hours with shaking. An amount of 0.3 g of glucose was added at the start of incubation, and then 0.3 g of glucose was fed after every 12 hours.

[Table 1]

| Strain | Astaxanthin content ($\mu$g/g dry cell) | Relative ratio (%) of HDCO |
|---|---|---|
| NBRC 10129 | 230 | 6.6 |
| KNK 01 | 1240 | 8.1 |
| KNK 02-1 | 3800 | 20.7 |
| KNK 02-2 | 6850 | 15.3 |
| KNK-03 | 12570 | 16.6 |

[Preparation 1]

*Xanthophyllomyces dendrorhous* KNK-03 was inoculated into four test tubes each including 5 ml of YM medium (polypeptone 0.5%, yeast extract 0.3%, malt extract 0.3%, glucose 1.0%) and incubated at 20°C for 48 hours. The cultures were transferred to four 500-ml Sakaguchi flasks each including 50 ml of YM medium and incubated at 20°C for 48 hours. The cultures were then transferred to a 5000-ml jar fermenter including 2500 ml of a medium (ammonium phosphate 1.3%, potassium phosphate 0.7%, yeast extract 0.3%, glucose 1%) and incubated at 20°C. In this manner, a culture including cells containing astaxanthin and HDCO was obtained. During the incubation, the pH was controlled in the range of 4.4 to 5.6, and glucose was fed such that the dissolved oxygen concentration was controlled in the range of 30 to 80% of the saturation concentration.

[Preparation 2]

A 10-ml aliquot of the culture prepared in Preparation 1 was poured into each 50-my centrifugation tube and centrifuged. The supernatant was removed and a cell pellet was obtained. To the pellet, 25 g of glass beads having a diameter of 0.5 mm and 25 ml of acetone were added, and the tube was sealed. Thereafter, the cells were disrupted using a multi-beads shocker (produced by Yasui Kikai Corp.) and the tube was centrifuged. The supernatant acetone phase was removed and the solvent was evaporated under reduced pressure. As a result, a cell extract containing astaxanthin and HDCO was obtained. The astaxanthin concentration of the cell extract was 6.1 mg/g.

[Preparation 3]

A glass column ($\phi$ 40 mm $\times$ 600 mm) filled with Silica gel 60 (produced by Merck) was equilibrated with a mixed solvent (hexane/acetone = 3/1), and the cell extract obtained in Preparation 2 was applied to the column. The cell extract was eluted with the same solvent and red fractions were combined. The solvent was evaporated under reduced pressure. As a result, a partially purified product containing astaxanthin and HDCO was obtained. The astaxanthin concentration of the cell extract was 53 mg/g.

[Preparation 4]

*Xanthophyllomyces dendrorhous* KNK-01 was inoculated into a test tube including 5 ml of YM medium (polypeptone 0.5%, yeast extract 0.3%, malt extract 0.3%, glucose 1.0%) and incubated at 20°C for 48 hours with shaking. A 0.6-ml aliquot of the culture was inoculated into 30 ml of a medium containing 1.3% ammonium phosphate, 0.7% potassium phosphate, 0.6% succinic acid, and 0.3% yeast extract (pH 5.4) and incubated in a 500-ml Sakaguchi flask at 20°C for 180 hours with shaking. As a result, a culture of cells containing astaxanthin and HDCO was obtained. An amount of 0.3 g of glucose was added as a carbon source at the start of incubation, and 0.3 g of glucose was fed every 12 hours after consumption of glucose.

[Preparation 5]

A 50-ml aliquot of the culture prepared in Preparation 4 was poured into each 50-ml centrifugation tube and centrifuged. The supernatant was removed and a cell pellet was obtained. To the pellet, 25 g of glass beads having a diameter of 0.5 mm and 25 ml of acetone were added, and the tube was sealed. Thereafter, the cells were disrupted using the multi-beads shocker (produced by Yasui Kikai Corp.) and the tube was centrifuged. The supernatant acetone phase was removed and the solvent was evaporated under reduced pressure. As a result, a cell extract containing astaxanthin and HDCO was obtained. The astaxanthin concentration of the cell extract was 5.6 mg/g.

[Preparation 6]

A microorganism was grown in the same manner as in Preparation 4, except that the microorganism was *Xanthophyllomyces dendrorhous* KNK-02-1. As a result, a culture of cells containing astaxanthin and HDCO was obtained.

[Example 1] Sulfuric acid treatment on *Xanthophyllomyces dendrorhous* KNK-03 cell extract

To airtight test tubes, 10-mg aliquots of the cell extract obtained in Preparation 2 were added. Then, 3-my portions of sulfuric acid aqueous solutions having concentrations shown in the following Table 2 were added to the respective tubes, and the contents were stirred. After the stirring, the solutions were assayed for pH using an F-22 pH meter (Horiba, Ltd.).

Each tube was shaken for two hours at 30°C, 50°C, or 70°C, and then 2 ml of chloroform was added thereto. The cell extract therein was dissolved by stirring. Each of the solutions was centrifuged to remove the aqueous phase, and the chloroform phase containing the treated cell extract was recovered. Each recovered phase was diluted to a 1/10 concentration with acetone and subjected to HPLC analysis to determine the relative ratio of HDCO to astaxanthin in the treated cell extract. The relative ratio of HDCO to astaxanthin was determined by the following equation:

$$\text{(Relative ratio (\%) of HDCO to astaxanthin)} = \{\text{(Peak area for HDCO)} / \text{(Peak area for astaxanthin)}\} \times 100.$$

The HPLC analysis was carried out under the following conditions.
Column: YMC Carotenoid (4.6 × 250 mm; YMC)
Column temperature: 20°C
Mobile phase: solution A (methanol/methyl-t-butyl ether/1% phosphoric acid aqueous solution = 82/15/3) and solution B (methanollmethyl-t-butyl ether/water/phosphoric acid = 7/90/3/0.03), the mobile phase was run at a flow rate of 1.0 ml/min under the following conditions:

after sample injection,

0 to 30 minutes: 100% solution A;
30 to 90 minutes: linear gradient from 100% solution A to 100% solution B; and
90 to 95 minutes: 100% solution B.

Astaxanthin was detected approximately 13 minutes after the start of the analysis, and HDCO was detected approximately 80 minutes after the start of the analysis under the above conditions.

Table 2 shows the results.

[Table 2]

| Concentration (N) of added sulfuric acid | pH after sulfuric acid addition | Relative ratio (%) of HDCO | | |
|---|---|---|---|---|
| | | Treatment at 30°C | Treatment at 50°C C | Treatment at 70°C |
| 0 | 6.4 | 15.2 | 15.2 | 15.1 |
| 0.1 | 1.4 | 14.9 | 14.7 | 13.9 |
| 0.5 | 0.8 | 14.8 | 14.0 | 12.5 |
| 1.0 | 0.6 | 14.5 | 13.1 | 11.0 |

(continued)

| Concentration (N) of added sulfuric acid | pH after sulfuric acid addition | Relative ratio (%) of HDCO | | |
|---|---|---|---|---|
| | | Treatment at 30°C | Treatment at 50°C C | Treatment at 70°C |
| 2.0 | 0.3 | 14.0 | 11.9 | 9.2 |
| Before treatment | - | 15.2 | | |

Table 2 demonstrates that the relative ratio of HDCO to astaxanthin in the *Xanthophyllomyces dendrorhous* KNK-03 cell extract containing astaxanthin and HDCO was reduced by contacting the cell extract with sulfuric acid.

[Example 2] Hydrochloric acid treatment on *Xanthophyllomyces dendrorhous* KNK-03 cell extract

The same procedures as in Example 1 were performed, except that hydrochloric acid was used instead of sulfuric acid. Table 3 shows the results.
[Table 3]

| Concentration (N) of added hydrochloric acid | pH after hydrochloric acid addition | Relative ratio (%) of HDCO | | |
|---|---|---|---|---|
| | | Treatment at 30°C | Treatment at 50°C | Treatment at 70°C |
| 0 | 6.4 | 15.2 | 15.2 | 15.1 |
| 0.5 | 0.9 | 15.0 | 14.2 | 12.6 |
| 1.0 | 0.6 | 14.6 | 13.3 | 11.8 |
| 2.0 | 0.4 | 14:2 | 12.6 | 10.0 |
| Before treatment | - | 15.2 | | |

Table 3 demonstrates that the relative ratio of HDCO to astaxanthin in the *Xanthophyllomyces dendrorhous* KNK-03 cell extract containing astaxanthin and HDCO was reduced by contacting the cell extract with hydrochloric acid. In addition, the results in Tables 2 and 3 show that the effect of the present invention was successfully produced regardless of the type of the acidic medium.

[Example 3] Sulfuric acid treatment on partially purified *Xanthophyllomyces dendrorhous* KNK-03 cell extract

The same procedures as in Example 1 were performed, except that the partially purified product obtained in Preparation 3 was used instead of the cell extract obtained in Preparation 2. Table 4 shows the results.
[Table 4]

| Concentration (N) of added sulfuric acid | pH after sulfuric acid addition | Relative ratio (%) of HDCO | | |
|---|---|---|---|---|
| | | Treatment at 30°C | Treatment at 50°0 | Treatment at 70°C |
| 0 | 6.7 | 9.3 | 9.3 | 9.2 |
| 0.5 | 0.6 | 8.9 | 8.7 | 7.9 |
| 1.0 | 0.4 | 8.4 | 8.0 | 6.5 |
| 2.0 | 0.1 | 7.9 | 7.1 | 4.7 |
| Before treatment | - | 9.3 | | |

Table 4 demonstrates that the relative ratio of HDCO to astaxanthin in the partially purified product of the *Xanthophyllomyces dendrorhous* KNK-03 cell extract containing astaxan-uhin and HDCO was reduced by contacting the partially purified cell extract with sulfuric acid.
The results in Tables 2 and 4 show that the method of the present invention is effective regardless of the concentrations of astaxanthin and HDCO in the composition containing these compounds.

[Example 4] Sodium hydroxide treatment on *Xanthophyllomyces dendrorhous* KNK-03 cell extract

The same procedures as in Example 1 were performed, except that sodium hydroxide was used instead of sulfuric acid. Table 5 shows the results.
[Table 5]

| Concentration (N) of added sodium hydroxide | pH after sodium hydroxide addition | Relative ratio (%) of HDCO | | |
|---|---|---|---|---|
| | | Treatment at 30°C | Treatment at 50°C | Treatment at 70° C |
| 0 | 6.4 | 15.2 | 15.2 | 15.1 |
| 0.1 | 12.9 | 15.0 | 14.7 | 14.2 |
| 0.5 | 13.4 | 14.7 | 14.5 | 13.8 |
| 1.0 | 13.7 | 14.3 | 13.9 | 12.7 |
| 2.0 | 13.9 | 13.9 | 11.4 | 9.7 |
| Before treatment | - | 15.2 | | |

Table 5 demonstrates that the treatment using sodium hydroxide reduces the relative ratio of HDCO to astaxanthin in the cell extract.

[Example 5] Potassium hydroxide treatment on *Xanthophyllomyces dendrorhous* KNK-03 cell extract

The same procedures as in Example 1 were performed, except that potassium hydroxide was used instead of sulfuric acid. Table 6 shows the results.
[Table 6]

| Concentration (N) of added potassium hydroxide | pH after potassium hydroxide addition | Relative ratio (%) of HDCO | | |
|---|---|---|---|---|
| | | Treatment at 30°C | Treatment at 50°C | Treatment at 70 °C |
| 0 | 6.4 | 15.2 | 15.2 | 15,1 |
| 0.5 | 13.0 | 14.9 | 14.6 | 14.1 |
| 1.0 | 13.5 | 14.6 | 14.4 | 13.0 |
| 2.0 | 13.7 | 14.2 | 12.0 | 10.5 |
| Before treatment | - | 15.2 | | |

Table 6 demonstrates that the treatment using potassium hydroxide reduces the relative ratio of HDCO to astaxanthin in the cell extract. In addition, the results in Tables 5 and 6 show that the effect of the present invention was successfully produced regardless of the type of the basic medium.

[Example 6] Sodium hydroxide treatment on partially purified *Xanthophyllomyces dendrorhous* KNK-03 cell extract

The same procedures as in Example 4 were performed, except that the partially purified product obtained in Preparation 3 was used instead of the cell extract obtained in Preparation 2. Table 7 shows the results.
[Table 7]

| Concentration (N) of added sodium hydroxide | pH after sodium hydroxide addition | Relative ratio (%) of HDCO | | |
|---|---|---|---|---|
| | | Treatment at 30°C | Treatment at 50°C | Treatment at 70°C |
| 0 | 6.7 | 9.3 | 9.3 | 9.2 |
| 0.5 | 13.6 | 9.2 | 9.0 | 8.6 |
| 1.0 | 13.8 | 8.8 | 8.5 | 7.7 |
| 2.0 | 14.0 | 7.9 | 7.1 | 6.0 |
| Before treatment | - | 9.3 | | |

Table 7 demonstrates that the relative ratio of HDCO to astaxanthin in the partially purified product of the *Xanthophyllomyces dendrorhous* KNK-03 cell extract containing astaxanthin and HDCO was reduced by contacting the partially purified cell extract with sodium hydroxide.

The results in Tables 5 and 7 show that the method of the present invention is effective regardless of the concentrations of astaxanthin and HDCO in the composition containing these compounds.

[Example 7] Sulfuric acid treatment on *Xanthophyllomyces dendrorhous* KNK-01 cell extract

The same procedures as in Example 1 were performed, except that the cell extract obtained in Preparation 5 was used instead of the cell extract obtained in Preparation 2. Table 8 shows the results.

[Table 8]

| Concentration (N) of added sulfuric acid | pH after sulfuric acid addition | Relative ratio (%) of HDCO | | |
|---|---|---|---|---|
| | | Treatment at 30°C | Treatment at 50°C | Treatment at 70°C |
| 0 | 5.9 | 8.1 | 8.0 | 8.0 |
| 0.5 | 0.8 | 7.9 | 7.7 | 7.1 |
| 1.0 | 0.5 | 7.5 | 7.0 | 6.2 |
| 2.0 | 0.3 | 6.8 | 6.1 | 5.3 |
| Before treatment | - | 8.1 | | |

Table 8 demonstrates that the relative ratio of HDCO to astaxanthin in the *Xanthophyllomyces dendrorhous* KNK-01 cell extract containing astaxanthin and HDCO was reduced by contacting the cell extract with sulfuric acid.

The results in Tables 2 and 8 show that the method of the present invention is effective regardless of the strain of origin of the composition containing astaxanthin and HDCO.

[Example 8] Sulfuric acid treatment on *Xanthophyllomyces dendrorhous* KNK-03 cell

Sulfuric acid was added to aliquots of the culture obtained in Preparation 1 to final concentrations shown in Table 9. After the addition of sulfuric acid, the resultant cultures were assayed for pH in the same manner as in Example 1. The results are shown in Table 9. Next, 10-ml aliquots of the cultures were poured into airtight glass vessels and each of them was stirred for two hours at 30°C, 50°C, or 70°C. After this two-hour treatment, 0.05-ml aliquots of the cultures were poured into 2-ml airtight polypropylene tubes and centrifuged to sediment cells. The supernatant was removed from each tube and the cells were resuspended in 1 ml of water and sedimented again by centrifugation. The supernatant was removed, and 1 g of glass beads ($\phi$ 0.5 mm) and 1 ml of acetone were added to each tube. The cells were then disrupted using a multi-beads shocker (produced by Yasui Kikai Corp.). After the disruption, the contents of each tube were filtered through a filter (Cosmonice Filter S, 0.45 $\mu$m, Millipore), and the filtrate was subjected to the HPLC analysis. The HPLC analysis and the calculation of the relative ratio of HDCO to astaxanthin were carried out in the same manner as in Example 1. Table 9 shows the results.

[Table 9]

| Concentration (N) of added sulfuric acid | pH after sulfuric acid addition | Relative ratio (%) of HDCO | | |
|---|---|---|---|---|
| | | Treatment at 30°C | Treatment at 50°C | Treatment at 70°C |
| 0 | 6.4 | 15.6 | 15.6 | 15.5 |
| 0.5 | 1.2 | 15.2 | 14.8 | 14.2 |
| 1.0 | 0.6 | 14.0 | 13.6 | 12.4 |
| 2.0 | 0.2 | 13.4 | 12.8 | 11.8 |
| Before treatment | - | 15.6 | | |

[Example 9] Sodium hydroxide treatment on *Xanthophyllomyces dendrorhous* KNK-03 cell

The same procedures as in Example 8 were performed, except that sodium hydroxide was used instead of sulfuric acid. Table 10 shows the results.
[Table 10]

| Concentration (N) of added sodium hydroxide | pH after sodium hydroxide addition | Relative ratio (%) of HDCO | | |
|---|---|---|---|---|
| | | Treatment at 30°C | Treatment at 50°C. | Treatment at 70°C |
| 0 | 6.4 | 15.6 | 15.6 | 15.5 |
| 0.2 | 9.0 | 15.5 | 15.3 | 14.9 |
| 0.5 | 10.9 | 15.2 | 14.8 | 14.5 |
| 1.0 | 13.1 | 14.0 | 14.5 | 11.7 |
| Before treatment | - | 15.6 | | |

[Example 10] Sulfuric acid treatment on *Xanthophyllomyces dendrorhous* KNK-03 cell

Into airtight glass vessels, 50-ml aliquots of the culture obtained in Preparation 1 were poured, and sulfuric acid was added to control the pH to 7.0 or 3.0. The vessels were sealed and the contents were stirred in a water-bath at 70°C. After 0 hours, 12 hours, 24 hours, and 48 hours, 0.05 ml portions were sampled from the vessels and the relative ratio of HDCO to astaxanthin in the cells was calculated in the same manner as in Example 8. Table 11 shows the results.
[Table 11]

| Treatment period (hr) | Relative ratio (%) of HDCO | |
|---|---|---|
| | pH 7.0 | pH 3.0 |
| 0 | 15.6 | 15.6 |
| 12 | 15.6 | 15.5 |
| 24 | 15.5 | 15.3 |
| 48 | 15.5 | 15.0 |
| Before treatment | 15.6 | |

[Example 11] Sulfuric acid treatment on *Xanthaphyllomyces dendrorhous* KNK-02-1 cell

Into airtight glass vessels, 50-ml aliquots of the culture obtained in Preparation 6 were poured, and sulfuric acid was added to control the pH to 7.0 or 1.0. The vessels were sealed and the contents were stirred in a water-bath at 70°C. After 0 hours, 3 hours, and 6 hours, 0.05 ml portions were sampled from the vessels and the relative ratio of HDCO to astaxanthin in the cells was calculated in the same manner as in Example 8.

Table 12 shows the results.

[Table 12]

| Treatment period (hr) | Relative ratio (%) of HDCO | |
|---|---|---|
| | pH 7.0 | pH 1.0 |
| 0 | 20.3 | 20.3 |
| 3 | 20.3 | 17.1 |
| 6 | 20.2 | 13.4 |
| Before treatment | 20.3 | |

The results in Tables 9 to 12 show that the effect of the present invention is successfully produced even when the composition containing astaxanthin and HDCO is cells of *Xanthophyllomyces dendrorhous.*

**Claims**

1. A method for producing an astaxanthin-containing composition, comprising:

   contacting a composition containing astaxanthin and 3-hydroxy-3',4'-didehydro-β,ψ-caroten-4-one (HDCO) with an acidic medium having a pH of 3 or less and/or a basic medium having a pH of 9 or greater to reduce a relative ratio of HDCO to astaxanthin in the composition.

2. The method according to claim 1,
   wherein the composition containing astaxanthin and HDCO is at least one selected from the group consisting of cells capable of producing astaxanthin, parts of the cells, extracts of the cells, and partially purified products of these.

3. The method according to claim 2,
   wherein the cells capable of producing astaxanthin are cells of a microorganism of the genus *Xanthophyllomyces, Brevundimonas, Haematococcus, Chlamydomonas, Monoraphidium, Erythrobaeter, Agrobacterium,* or *Paracoccus,* or a microorganism of Labyrinthulea.

4. The method according to claim 2,
   wherein the cells capable of producing astaxanthin are cells of a microorganism of the genus *Xanthophyllomyces.*

5. The method according to claim 4,
   wherein the microorganism cells of the genus *Xanthophyllomyces* have an astaxanthin content of not less than 2000 μg/g dry cell weight.

6. The method according to any one of claims 1 to 5,
   wherein the composition containing astaxanthin and HDCO has an relative ratio of HDCO to astaxanthin of not less than 1%.

7. The method according to any one of claims 1 to 6,
   wherein the acidic medium has a pH of 2 or less.

8. The method according to any one of claims 1 to 6,
   wherein the basic medium has a pH of 10 or greater.

9. The method according to any one of claims 1 to 8,
   wherein the relative ratio of HDCO to astaxanthin is reduced by 0.1 percentage points or more through the contact with the acidic medium and/or the basic medium.

10. A feed, a food, a food additive, or a medicament, comprising an astaxanthin-containing composition obtained by the method according to any one of claims 1 to 9.

<table>
<tr><td colspan="2" align="center">**INTERNATIONAL SEARCH REPORT**</td><td>International application No.<br>PCT/JP2010/005036</td></tr>
</table>

A. CLASSIFICATION OF SUBJECT MATTER
*C12P23/00*(2006.01)i, *A23K1/16*(2006.01)i, *A23L1/30*(2006.01)i, *A61P3/00* (2006.01)i, *A61K31/01*(2006.01)n, *A61K31/122*(2006.01)n, *A61K35/74* (2006.01)n, *C12R1/01*(2006.01)n, *C12R1/645*(2006.01)n
According to International Patent Classification (IPC) or to both national classification and IPC

B. FIELDS SEARCHED

Minimum documentation searched (classification system followed by classification symbols)
C12P23/00, A23K1/16, A23L1/30, A61P3/00, A61K31/01, A61K31/122, A61K35/74, C12R1/01, C12R1/645

Documentation searched other than minimum documentation to the extent that such documents are included in the fields searched
Jitsuyo Shinan Koho          1922–1996     Jitsuyo Shinan Toroku Koho     1996–2010
Kokai Jitsuyo Shinan Koho     1971–2010     Toroku Jitsuyo Shinan Koho     1994–2010

Electronic data base consulted during the international search (name of data base and, where practicable, search terms used)
CA/BIOSIS/MEDLINE/WPIDS(STN), JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
|---|---|---|
| A/X | JP 6-506122 A  (Gist-Brocades N.V.),<br>14 July 1994 (14.07.1994),<br>& US 5648261 A          & US 5879927 A<br>& EP 551676 A1          & WO 1993/012249 A1 | 1-9/10 |
| A/X | JP 8-228765 A  (Kaneka Corp.),<br>10 September 1996 (10.09.1996),<br>(Family: none) | 1-9/10 |
| A/X | JP 8-214870 A  (Kaneka Corp.),<br>27 August 1996 (27.08.1996),<br>(Family: none) | 1-9/10 |
| A/X | JP 7-099932 A  (Nippon Suisan Kaisha, Ltd.),<br>18 April 1995 (18.04.1995),<br>(Family: none) | 1-9/10 |

☐ Further documents are listed in the continuation of Box C.     ☐ See patent family annex.

| | |
|---|---|
| *   Special categories of cited documents:<br>"A"  document defining the general state of the art which is not considered to be of particular relevance<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| | |
|---|---|
| Date of the actual completion of the international search<br>12 October, 2010 (12.10.10) | Date of mailing of the international search report<br>19 October, 2010 (19.10.10) |
| Name and mailing address of the ISA/<br>Japanese Patent Office | Authorized officer |
| Facsimile No. | Telephone No. |

Form PCT/ISA/210 (second sheet) (July 2009)

14

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3202018 B
- JP 2920818 B